# EUROPEAN PATENT SPECIFICATION

(11) **EP 4 192 559 B1**
(45) Date of publication and mention of the grant of the patent: **29.10.2025**
(21) Application number: 20780441.0
(22) Date of filing: 09.09.2020
(51) Int. Cl.: A61M 25/00, A61M 39/12

(54) **PORT-CATHETER ASSEMBLY GRIPPING, CUTTING AND CONNECTION TOOL**
GREIF-, SCHNEIDE- UND VERBINDUNGSWERKZEUG FÜR PORTKATHETERANORDNUNG
OUTIL DE PRÉHENSION, DE COUPE ET DE RACCORDEMENT D'ENSEMBLE ORIFICE-CATHÉTER

(43) Date of publication of application: 14.06.2023
(73) Proprietor: Bard Peripheral Vascular, Inc., Franklin Lakes, NJ 07417 (US)
(72) Inventor: EVANS, John, G., South Jordan, UT 84095 (US)
(74) Representative: Hoffmann Eitle
(86) International application number: PCT/US2020/049874
(87) International publication number: WO 2022/055481

(56) References cited:
- WO-A1-2019/040801
- US-A- 5 368 561
- US-A1- 2010 049 116

## Description

### BACKGROUND

When placing catheters, e.g. Central Vascular Catheters (CVC) or Peripherally Inserted Central Catheters (PICC), the exact location of the distal tip within the vasculature can be essential to the efficacy of the catheter system and the treatment administered. For example, if the distal tip is not inserted far enough, the efficacy of the treatment is reduced. If the distal tip is inserted too far, the distal tip can cause cardiac arrhythmia. Catheters that are formed integrally with the port require estimating the length of the catheter prior to placement. Errors in estimating catheter length can be highly detrimental to the patient.

Proximally trimmable catheter port systems allow the catheter portion to be placed prior to trimming the catheter to an appropriate length. This can result in more accuracy in sizing the catheter relative to the target location and the port. Further, such port/catheter systems allow for the replacement of one of the port portion or the catheter portion without disturbing the positioning of the other portion. However, the catheter must then be attached to the port within the tissue pocket. Trimming the catheter and attaching the catheter to the port with a cathlock, all within the confined, wetted environment of a tissue pocket can be difficult. Such a process can require the manipulation of several tools to hold the catheter, hold the cathlock, trim the catheter, attach the catheter to the port, and finally lock the cathlock in place. This can lead to an increase risk of trauma to the insertion site due multiple tools being inserted at one time. Further repeated insertion of multiple tools can result in an increased risk of infection, and such a complex procedure can be time consuming.

US 2012/049116 A1 discloses a catheter assembly including a catheter having a proximal and distal end and at least one lumen extending between the ends. At least one end of the catheter is formed from a material that can be trimmed to achieve a selected length for the catheter. A tubular connector is telescoped over the catheter and a hub is joined to the tubular connector. Proximal portions of the hub are confined for connection to a medical apparatus. A cuff is mounted around the tubular connector or the catheter. The cuff is formed from a material that will permit or promote the growth of scar tissue for anchoring the catheter device at least on a semi-permanent basis in a patient.

### SUMMARY

Embodiments disclosed herein are directed to a cathlock placement and attachment tool for use with a proximally trimmable catheter and port systems. Advantageously, the tool provides a single tool configured to hold the catheter, hold the cathlock, trim the catheter, attach the catheter to the port, and lock the cathlock in place. This can mitigate repeated insertion of multiple tools and can expedite placing proximally trimmable port/catheter systems.

Disclosed herein is a stabilization and trimming tool for coupling a catheter to an access port including, a handle operatively coupled to a cradle and a clamping device, wherein the cradle is designed to hold a cathlock, and wherein the clamping device is designed to grip a catheter, and a catheter cutting mechanism coupled to the handle, wherein the catheter cutting mechanism is configured to be actuated following stabilization of the cathlock and the catheter by the handle.

In some embodiments, a first pressure applied to the handle applies pressure to the cradle, and wherein a second pressure greater than the first pressure applies pressure to both the cradle and the clamping device. The cradle defines a channel, an inner surface of the channel defines a profile that mirrors an outer profile of the cathlock. The inner surface of the channel defines a tapered shape. The inner surface of the channel defines a stepped portion configured to abut against a stepped portion of the cathlock. The clamping device includes a pair of clamping members covered by a rubber or silicone material. The catheter cutting mechanism is actuated by an actuator. The cradle includes a pair of jaws transitionable between an open position and a closed position, the jaws configured to retain the cathlock within the cradle in the closed position. A first jaw of the pair of jaws is rotatably coupled to a first edge of the cradle and a second jaw of the pair of jaws is rotatably coupled to a second edge of the cradle. The cradle defines a channel and includes a pair of grips transitionable between a retracted position and an extended position, the grips configured to retain the cathlock within the cradle in the extended position. The pair of grips are slidably engaged with the cradle along an axis extending perpendicular to an axis of the channel.

Also disclosed but not claimed is a method of attaching a catheter to an access port including, creating a tissue pocket in a patient and inserting the access port into the pocket, the access port including a stem, inserting the catheter into a vessel of the patient until a tip of the catheter is positioned at a desired location, sliding a cathlock over a proximal end of the catheter, gripping the cathlock and the catheter with a tool, removing a proximal portion of the catheter using the tool, inserting the stem into an open end of the catheter, and sliding the cathlock over the catheter to secure the cathlock and the catheter to the access port.

In the present invention, the stabilization and trimming tool comprises a handle operatively coupled to a cradle and a clamping device, wherein gripping the cathlock and the catheter comprises inserting the cathlock into the cradle, positioning the clamping device over the catheter, and actuating the handle to apply a gripping force to the cradle and the clamping device. The cradle defines a channel, an inner surface of the channel defines a profile that mirrors an outer profile of the cathlock. The inner surface of the channel defines a tapered shape. The inner surface of the channel defines a stepped portion configured to abut against a stepped portion of the cathlock. The tool further comprises a catheter cutting mechanism coupled to the handle, and wherein removing the proximal portion of the catheter comprises actuating the cutting mechanism after actuating the handle. The cutting mechanism includes a first cutting member rotatably coupled to the handle. The cutting mechanism includes a first cutting member and a second cutting member rotatably coupled to the handle in a scissor-like mechanism.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1A shows a left side view of an exemplary cathlock placement tool including a cradle, in accordance with embodiments disclosed herein.
FIG. 1B shows a plan view of an exemplary cathlock placement tool, in accordance with embodiments disclosed herein.
FIG. 1C shows a right side view of an exemplary cathlock placement tool, in accordance with embodiments disclosed herein.
FIG. 2A shows close up detail of an embodiment of a cradle, in accordance with embodiments disclosed herein.
FIG. 2B shows close up detail of an embodiment of a cradle in a closed position, in accordance with embodiments disclosed herein.
FIG. 2C shows close up detail of an embodiment of a cradle in an open position, in accordance with embodiments disclosed herein.
FIG. 2D shows close up detail of an embodiment of a cradle, in accordance with embodiments disclosed herein.
FIG. 3A shows close up detail of an embodiment of a cradle including a gripping mechanism in an open position, in accordance with embodiments disclosed herein.
FIG. 3B shows close up detail of an embodiment of a cradle including a gripping mechanism in a closed position, in accordance with embodiments disclosed herein.
FIG. 4A shows close up detail of an embodiment of a cradle including a cutting mechanism in a first position, in accordance with embodiments disclosed herein.
FIG. 4B shows close up detail of an embodiment of a cradle including a cutting mechanism in a second position, in accordance with embodiments disclosed herein.
FIG. 5A shows a cross-sectional view of a cathlock, in accordance with embodiments disclosed herein.
FIG. 5B shows a side view of a cathlock, in accordance with embodiments disclosed herein.
FIG. 5C shows a cross-sectional view of a cathlock, in accordance with embodiments disclosed herein.
FIG. 5D shows a side view of a cathlock, in accordance with embodiments disclosed herein.
FIGS. 5E-5F show perspective views of a cathlock, in accordance with embodiments disclosed herein.
FIGS. 6A-6E show an exemplary method of use for a cathlock placement tool, in accordance with embodiments disclosed herein.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein. The invention is defined in claim 1.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

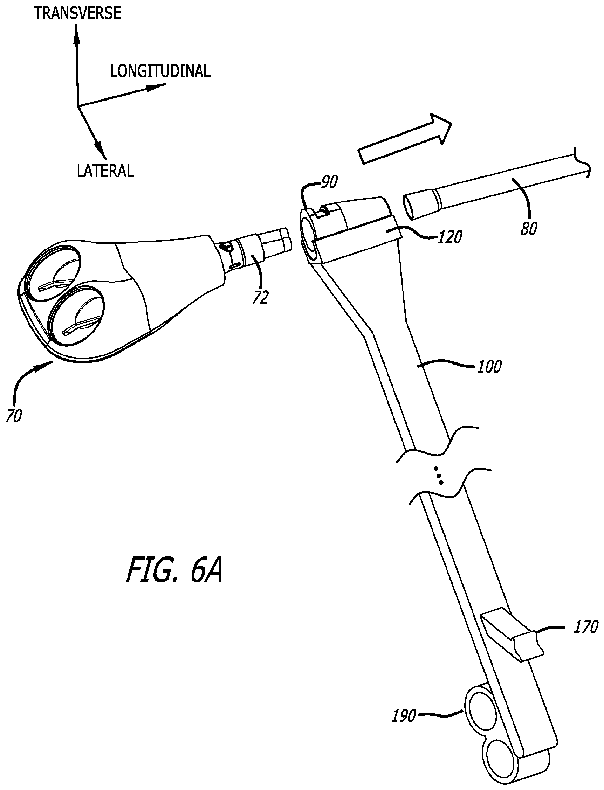

As used herein, and as shown in FIG. 6A, a longitudinal axis extends along an axial length of a catheter, a lateral axis extends normal to the longitudinal axis and a transverse axis extends normal to both the longitudinal and the lateral axes. As used herein a "taper" shape is a decrease in cross-sectional shape, or in diameter, along an axis. For example such as a conical or frusto-conical shape, or the like. As used herein "operatively coupled" can include gears, levers, mechanisms, biasing members, electrical motors, wiring, switches, power sources or the like, configured to operate a mechanism.

Unless defined otherwise, all technical and scientific terms used herein have the same meaning as commonly understood by those of ordinary skill in the art.

The present disclosure generally relates to a cathlock placement tool ("tool") 100 configured to stabilize a cathlock 90 relative to a proximal portion of a catheter 80, and trim a portion of the catheter 80 to facilitate coupling with a port 70, securing the catheter thereto with a cathlock 90.

Proximally trimmable catheters and port systems are configured to allow placement of a distal portion of the catheter within a vasculature of the patient and couple an access port, or similar medical device, to a proximal end. Advantageously, a distal tip of the catheter can be placed at a target location prior to trimming the proximal end to a correct length and coupling to the port 70. This mitigates miscalculations of catheter length from sizing and coupling catheter and port systems prior to placement. Further, the proximal end of the catheter 80 can be trimmed to fit a location of the port 70 already disposed within a tissue pocket. This can be of particular relevance to port catheter systems where the catheter 80 can be detached and replaced, while the port 70 remains in place within the tissue pocket. Detachable port/catheter systems can include a cathlock 90, or similar device, configured to secure the catheter 80 to a stem of the port 70.

Embodiments, disclosed herein are directed to a tool 100 configured to stabilize a cathlock device ("cathlock") 90 relative to a proximal portion of a catheter 80 and trim the proximal portion of the catheter 80 to a correct length for attachment to a subcutaneous access port ("port") 70. Advantageously, embodiments disclosed herein provide a single tool for stabilizing the catheter, stabilizing the cathlock, trimming the catheter, attaching the catheter to the port, and locking the cathlock in place.

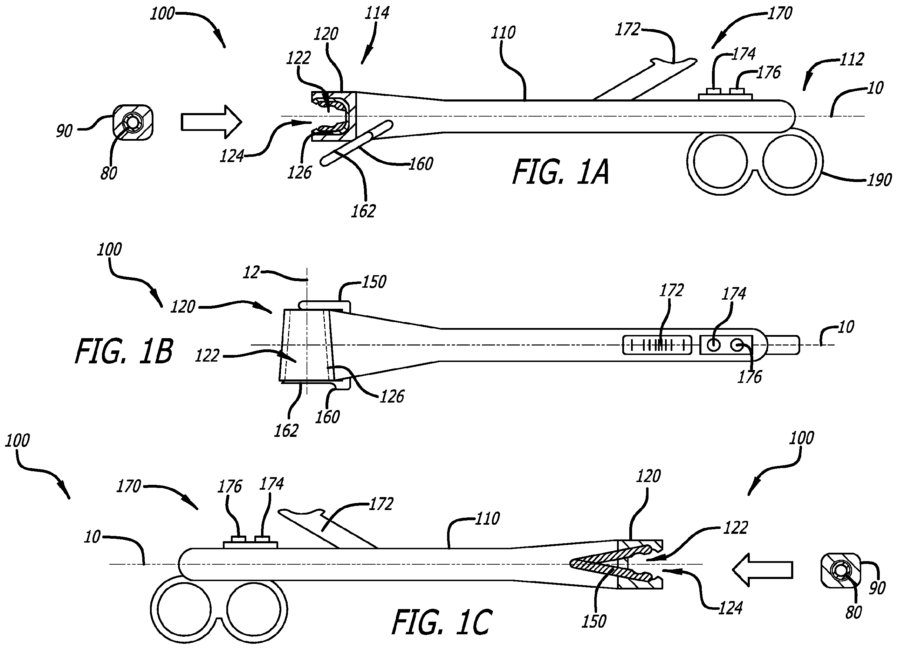

As shown in FIGS. 1A-1C the tool 100 includes an elongate handle 110 extending from a first end 112 to a second end 114 to define an axial length extending along axis 10. A cradle 120 can be disposed at the second end 114 of the handle 110 and is configured to retain a cathlock 90 therein. The cradle 120 can include a channel 122 defining a channel axis 12 extending perpendicular to the central axis 10. The cradle 120 can include an elongate opening 124 extending parallel to the channel axis 12 and configured to allow ingress or egress of the cathlock 90 to/from the channel 122.

An inner surface 126 of the channel 122 can define a profile that mirrors a profile of an outer surface of the cathlock 90, or portion thereof. As such a cathlock 90 can fit securely within the channel 122 to retain the cathlock 90 therein. In an embodiment, the inner surface 126 of the cradle can include a material or textured surface that can increase a friction coefficient between the cradle 120 and the cathlock 90, e.g. silicone, rubber, or the like. In an embodiment, the channel 122 can define a tapering inner surface 126 configured to restrict movement of the cathlock 90 relative to the cradle 120 in a first direction along the channel axis 12.

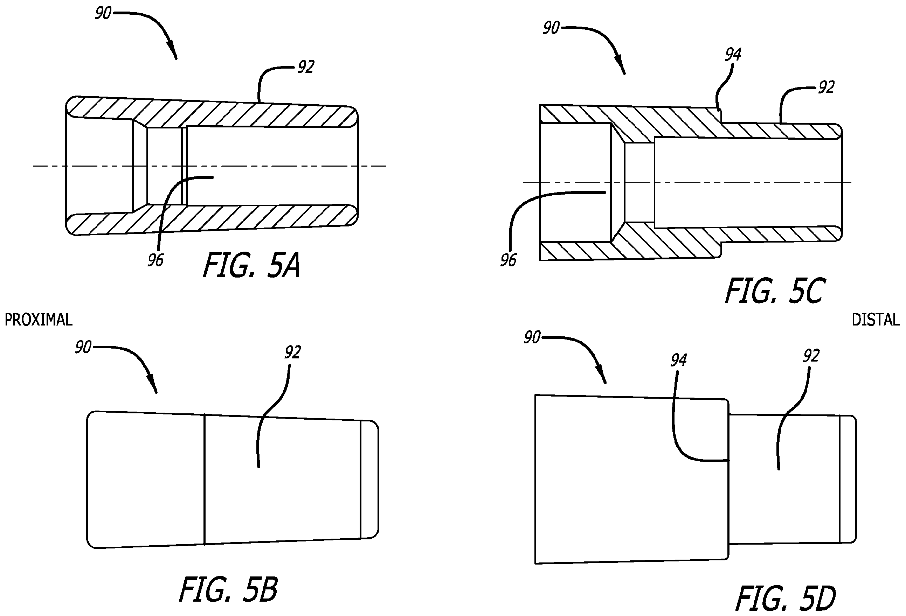

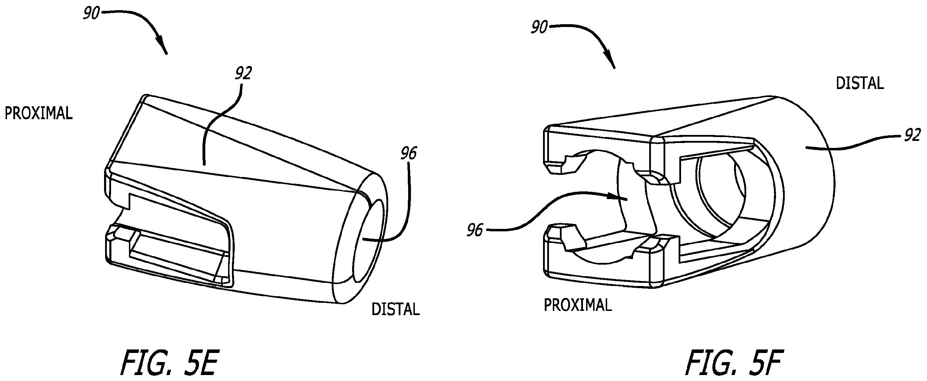

FIGS. 5A-5F show various views of exemplary cathlocks 90. As noted, the inner profile of the channel 122 can match that of the outer profile of the cathlock 90, as such the inner profile of the channel 122 can be configured to receive and retain one or more of the exemplary cathlocks 90 shown in FIGS. 5A-5F. It will be appreciated, however, that these exemplary cathlocks 90 are not intended to be limiting. In an embodiment, the cathlock 90 can define a substantially cylindrical shape extending along a longitudinal axis. A cross-sectional shape of the outer surface of the cathlock 90, i.e. a cross-sectional shape extending perpendicular to the longitudinal axis, can be circular, elliptical, rectangular, rounded rectangular, or combinations thereof. However it will be appreciated that the cathlock 90 can define any regular or irregular closed curve polygonal cross-sectional shape.

In an embodiment, the cathlock 90, or portion thereof, can define a tapering shape including a decreasing diameter between a proximal end and a distal end. In an embodiment, the outer surface 92 of the cathlock 90 can define one or more stepped portions 94 to define an abutment configured to engage an abutment surface of the channel 122 to restrict movement of the cathlock 90 relative to the cradle 120. The stepped portion 94 can extend annularly about a portion of the cathlock 90, about the longitudinal axis. In an embodiment, the outer surface of the cathlock can include one or more protrusions (not shown) to define an abutment configured to engage an abutment surface, or recess, of the channel 122 to restrict movement of the cathlock 90 relative to the cradle 120.

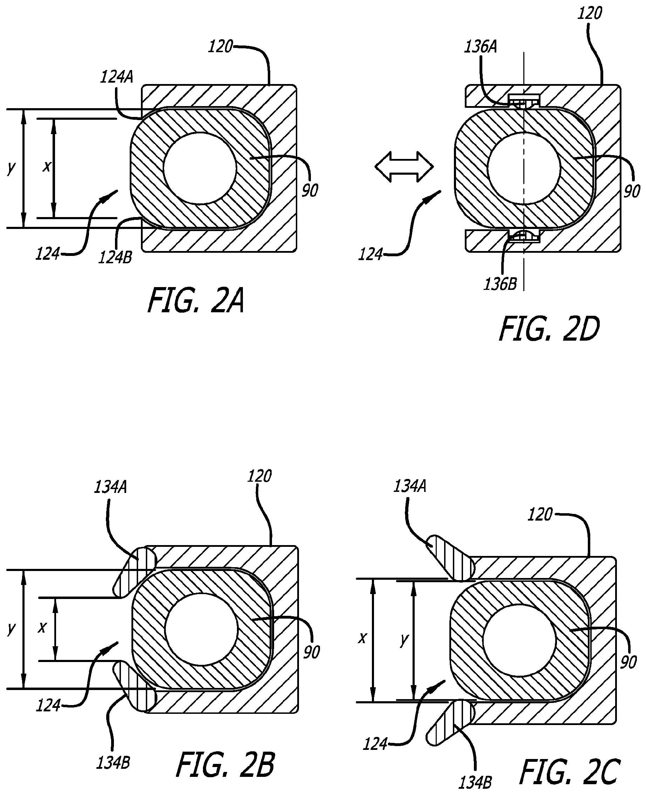

FIGS. 2A-2D show further embodiments of the cradle 120. As shown in FIG. 2A, in an embodiment, a width (x) of the opening 124 can be less than an outer cross-sectional diameter (y) of the cathlock 90. Further a material of the cradle 120 can include resilient mechanical properties. As such the cathlock 90 can be urged through opening 124, along an axis 10 of the tool 100, and the cradle 120 can flexibly deform slightly to allow the cathlock 90 to pass therethrough and be received within the channel 122. When the cathlock 90 is located within the channel 122, the cradle can resume the undeformed shape and retain the cathlock 90 therein. An edge of the opening, e.g. a top edge 124A or a bottom edge 124B can abut against an outer surface of the cathlock 90 to prevent the cathlock 90 from disengaging the cradle 120 prematurely. Similarly, the cathlock 90 can be withdrawn from the channel 122, through the opening 124 by urging the cathlock 90 along an axis 10 of the tool 100. This can cause the cradle to deform slightly to allow the cathlock 90 to pass through the opening 124, before returning to the undeformed shape and disengaging the cathlock 90.

As shown in FIGS. 2B-2C, the cradle 90 can further include one or more jaws 134 rotatably coupled to an edge of the opening 124 and transitionable between a closed position (FIG. 2B) and an open position (FIG. 2C). In an embodiment, a width (x) of the opening 124 can be the same or slightly larger than an outer diameter (y) of the cathlock 90. As such, in an open position, the cathlock 90 can slide through the opening 124 to be received within the channel 122. The cradle 120 can include a first jaw 134A, and a second jaw 134B disposed opposite the first jaw 134A across the opening 124. In a closed position, the jaws 134A, 134B, can rotate inwards towards the opening 124 to constrict the width (x) of the opening to a distance that is less than a diameter (y) of the cathlock 90, and retain the cathlock 90 within the channel 122. In an embodiment, the first jaw 134A and the second jaw 134B can contact each other in the closed position. In an open position, the jaws 134A, 134B, can rotate outwards away from the opening 124, opening up a width (x) of the opening 124, and allowing ingress/egress of the cathlock 90 to/from the channel 122.

In an embodiment, the jaws 134 can be operatively coupled to an actuator 170 disposed proximate a first end 112 of the handle 110. The actuator 170 is configured to allow a user to transition the jaws 134 between the open position and the closed position, as described in more detail herein. In an embodiment the jaws 134 can include a rubber, silicone, or similar material to increase a friction coefficient between the jaws 134 and the cathlock 90 and facilitate retaining the cathlock 90 within the channel 122.

As shown in FIG. 2D, in an embodiment, the cradle 90 can further include one or more grips 136, e.g. a first grip 136A and a second grip 136B, disposed within a wall of the channel 122 and transitionable between an extended position, e.g. first grip 136A, and a retracted position, e.g. 136B. To note, in an embodiment, the grips 136A, 136B can both be in the extended position or both be in the retracted position. The grips 136 can transition between the extended position and the retracted position along an axis that extends perpendicular to both the channel axis 12 and the direction of ingress/egress of the cathlock 90 from the channel 122, i.e. the axis 10 of the tool 100. In an extended position, the grips 136A, 136B, can impinge on an outer surface of the cathlock 90 and pressing the cathlock 90 against an opposite grip 136 and/or an opposite wall of the channel 122 and retaining the cathlock 90 within the channel 122. In a retracted position, the grips 136A, 136B, can be withdrawn into a recess within the wall of the channel 122, releasing the cathlock 90 and allowing ingress/egress of the cathlock 90 to/from the channel 122.

In an embodiment, the grips 136 can be operatively coupled to an actuator 170 disposed proximate a proximal end of the handle 110. The actuator 170 is configured to allow a user to transition the grips 136 between an extended position and a retracted position, as described in more detail herein. In an embodiment the grips 136 can include a rubber, silicone, or similar material to increase a friction coefficient between the grips 136 and the cathlock 90 and to facilitate retaining the cathlock 90 within the channel 122.

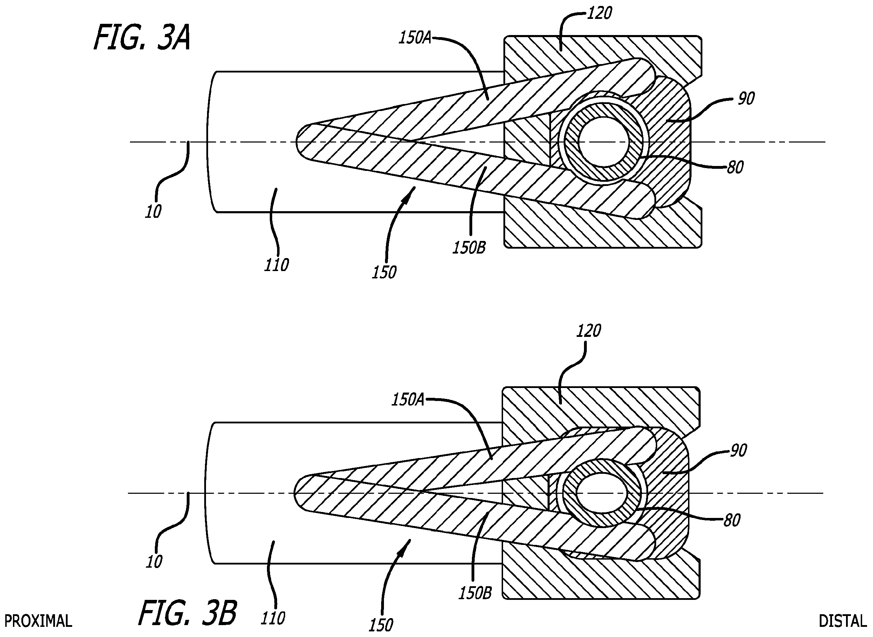

As shown in FIGS. 1A, 3A-3B, the tool 100 further includes a clamping device ("clamp") 150 operatively coupled with the tool 100, proximate the cradle 120, and configured to selectively retain a portion of the catheter 80 extending through the cradle 120, along the channel axis 12. The clamp 150 can transition between an open position (FIG. 3A) and a closed position (FIG. 3B). With the clamp 150 in the open position, a portion of the catheter 80 can pass unimpeded through the channel 122, along the channel axis 12. In the closed position, the clamp 150 can grasp a portion of the catheter 80 to inhibit movement of the catheter 80 relative to the tool 100.

In an embodiment, the clamp 150 can include a first clamping member 150A and a second clamping member 150B. One of the first clamping member 150A or the second clamping member 150B can be rotatably coupled with the tool to transition the clamp 150 between the open position and the closed position. In an embodiment, the clamp 150 can be operatively coupled to an actuator 170 to allow a user to transition the clamp 150 between the open position and the closed position. In an embodiment, the clamp 150 can include a material or textured surface that can increase a friction coefficient between the clamp 150 and the catheter 80, e.g. silicone, rubber, or the like. In an embodiment, the clamp 150 can include a biasing member, e.g. spring or the like, configured to bias the clamp 150 towards one of the open position or the closed position.

It will be appreciated that various clamping mechanisms are contemplated to fall within the scope of the present invention including sliding members, slidably engaged with the tool 100, wire members configured to encircle and tighten around the catheter 80, compression members configured to compress the catheter against a portion of the tool 100, combinations thereof, or the like.

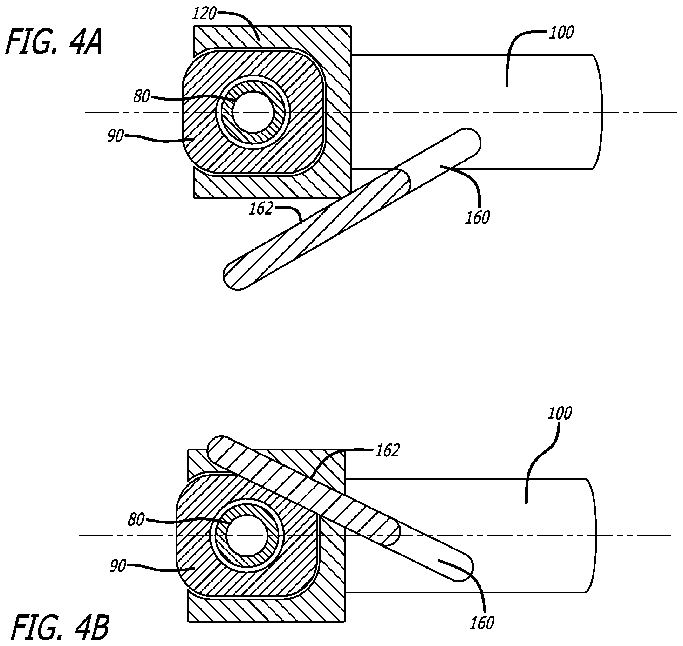

As shown in FIGS. 1A, 4A-4B, the tool 100 further includes a cutting member 150, operatively coupled with the tool 100 proximate the cradle 120 and configured to trim a portion of a catheter 80 extending through the channel 122. In an embodiment, the cutting member 160 can be rotatably coupled with the tool 100 such that a sharpened cutting edge 162 of the cutting member 160 can transition across the channel axis 12 between a first position (FIG. 4A) and a second position (FIG. 4B). In an embodiment, the cutting member 160 can be operatively coupled to an actuator 170 to allow a user to transition the cutting member 160 between the first position and the second position.

In an embodiment, the cutting member 160 can include a biasing member, e.g. spring or the like, configured to bias the cutting member 160 towards one of the first position or the second position. For example, a biasing member can bias the cutting member 160 towards the first position and the user can actuate an actuator 170 to transition the cutting member 160 to the second position, cutting a portion of the catheter 80 extending across the cutting member 160. When a user releases the actuator 170, the biasing member can transition the cutting member 160 from the second position to the first position.

In an embodiment, a biasing member can bias the cutting member 160 towards the second position. The cutting member 160 can be retained in the first position by a trigger. A user can actuate an actuator 170 to release the trigger and allow the biasing member to transition the cutting member 160 from the first position to the second position. Optionally an actuator 170 can be configured to reset the cutting member 160 from the second position to the first position such that the trigger can retain the cutting member 160 in the first position.

It will be appreciated that various cutting mechanisms 160 are contemplated to fall within the scope of the present invention including sliding cutting members, slidably engaged with the tool 100, wire members configured to encircle and tighten around the catheter 80 to cut through a portion thereof, scissor-like cutting members rotatably coupled with the tool and configured rotate in opposite directions relative to each other, combinations thereof, or the like.

As shown in FIGS. 1A-1C, in an embodiment, the tool 100 can include one or more actuators 170 configured to actuate one of the jaws 134, grips 136, clamping mechanism 150, cutting member 160, or combinations thereof. In an embodiment, the one or more actuators 170 can be a lever (e.g. first actuator 172), button (e.g. second actuator 174, third actuator 176), switch, rocker switch, slider, spin nut, combinations thereof, or the like. In an embodiment, a first actuator 172 can be operatively coupled to one of the jaws 134, the grips 136, or combinations thereof. A second actuator 174 can be operatively coupled to a clamping mechanism 150 and a third actuator 176 can be operatively coupled to a cutting mechanism 160.

In an embodiment, a first actuator 172 can be operatively coupled to one of the jaws 134 or the grips 136, and the clamping mechanism 150. For example, the first actuator 172 can be moved from a first position to a second position to actuate one of the jaws 134 or the grips 136, and can then be moved to a third position to actuate the clamping mechanism 150. A second actuator 174 can be operatively coupled to a cutting mechanism 160. Optionally, the tool 100 can include a finger loop 190 or similar structure configured to facilitate grasping and manipulating the tool 100.

As used herein "operatively coupled" can include gears, levers, mechanisms, biasing members, springs, electrical motors, wiring, switches, power sources or the like, configured to operate a mechanism. For example, an actuator 170 can include a lever rotatably coupled to a first end of an actuator arm (not shown), which is disposed within an interior of the handle 110. A second end of the arm, opposite the first end, can be coupled with one of the jaws 134, grips 136, clamping mechanism 150, cutting member 160, or combinations thereof. Actuating the actuator 170 can move the arm within the interior of the handle 110 to actuate one of the jaws 134, grips 136, clamping mechanism 150, cutting member 160, or combinations thereof, as described herein, e.g. transition the jaws between open and closed, or the grips between extended and retracted, etc. In an embodiment, an actuator 170 can include a switch configured to close an electrical circuit to actuate a motor, servomechanism, or similar device. The motor can be disposed within an interior of the handle 110 and configured to actuate one of the jaws 134, grips 136, clamping mechanism 150, cutting member 160, or combinations thereof as described herein, e.g. transition the jaws between open and closed, or the grips between extended and retracted, etc.

As shown in FIGS. 6A-6E, in an exemplary method of use, a cathlock placement tool 100, as described herein, can retain a cathlock 90, stabilize and trim a proximal portion of the catheter 80, couple the catheter to a stem 72 of the port 70, and secure the catheter 80 thereto with the cathlock 90. In an example, a distal portion of a catheter 80 can be placed at a target location within a vasculature of the patient. A proximal end of the catheter 80 can extend from the incision site adjacent a tissue pocket with the port 70 disposed therein.

As shown in FIG. 6A, a cathlock 90 can be disposed within a cradle 120 of the tool 100. As described herein, the shape of the channel 122 can mirror an outer profile of the cathlock 90 such that the cathlock 90 fits securely within the cradle 120. In an example, a user can actuate one of a jaw 134 or grip 136, using an actuator 170, to retain the cathlock 90 within the channel 122. In an example, a user can actuate a first actuator 172 from a first position to a second position to retain the cathlock 90 within the channel 122. A user can then manipulate the tool 100 to thread a proximal portion of the catheter 80 through a lumen 96 of the cathlock 90 that is disposed within the cradle 120.

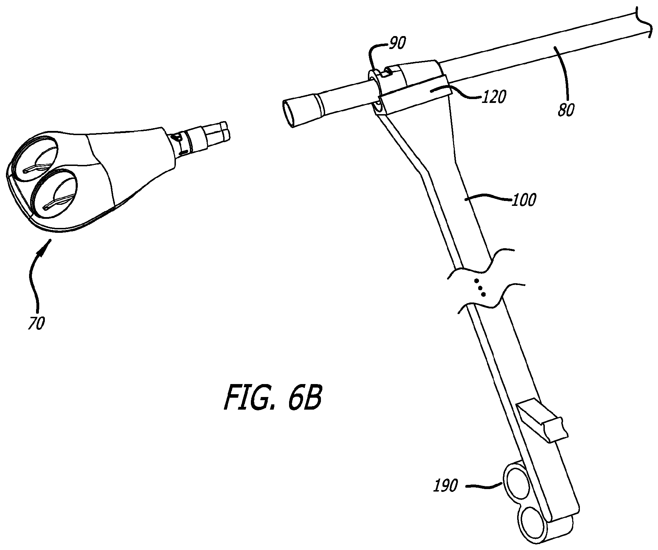

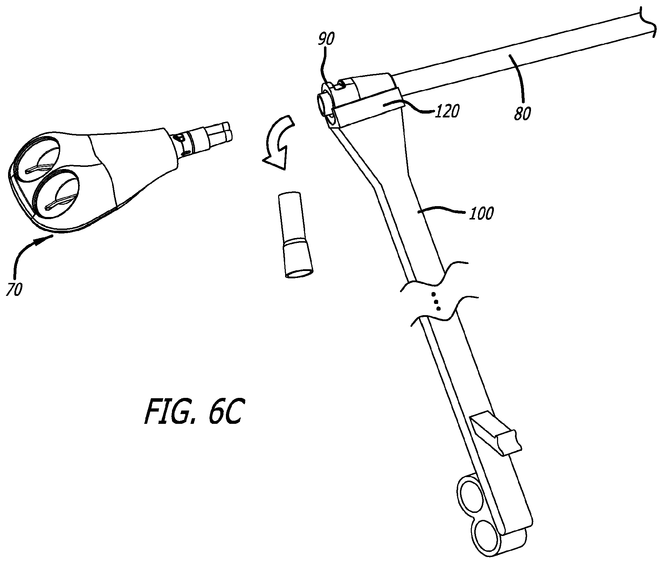

As shown in FIG. 6B, the user can then manipulate the tool 100 position the cathlock 90 at a suitable position along a length of the catheter 90. As shown in FIG. 6C, the user can then actuate one or more actuators 170 to actuate a gripping mechanism 150 to stabilize the catheter 80 relative to the cathlock 90 and the tool 100. In an example, a user can actuate the first actuator 172 to a third position to stabilize the catheter 80 relative to the cathlock 90 and the tool 100.

The user can then actuate an actuator 170 to actuate a cutting mechanism 160 to trim the proximal portion of the catheter 80 to a correct length. For example, a user can actuate a second actuator 174 to actuate a cutting mechanism 160 to trim the proximal portion of the catheter 80 to a correct length. Advantageously, since the cathlock 90 is engaged with the tool 100, if the cathlock 90 is correctly positioned relative to the catheter 80 and the catheter 80 is then stabilized relative to the cathlock 90 by the gripping mechanism 150, the cutting member 160 will be correctly positioned to trim the proximal portion of the catheter 80 to the correct length.

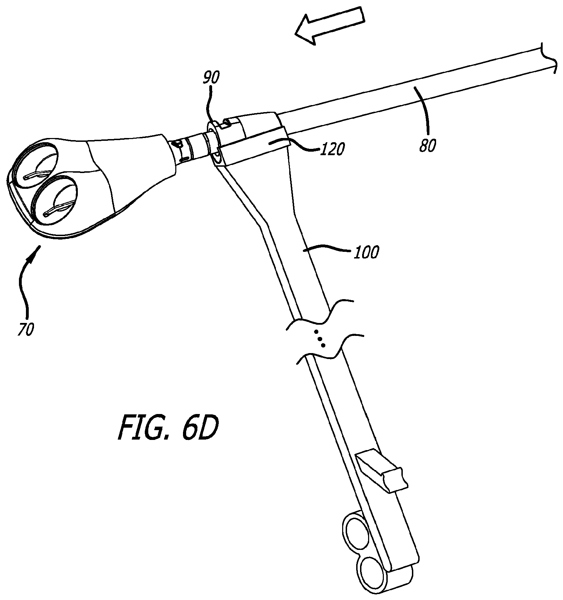

As shown in FIG. 6D, the tool 100 with both the cathlock 90 and the catheter 80 retained therein, can then be used to urge the trimmed catheter 80 onto the port stem 72, and secure the cathlock 90 in place, to secure catheter 80 to the port 70. Advantageously, the tool 100 can secure both the catheter 80 and the cathlock 90 simultaneously, allowing a user to manipulate the catheter 80 and cathlock 90 more easily within the tissue pocket. Further the tool 100 provides increased leverage to hold the catheter 80/cathlock 90 and urge onto the port stem 72.

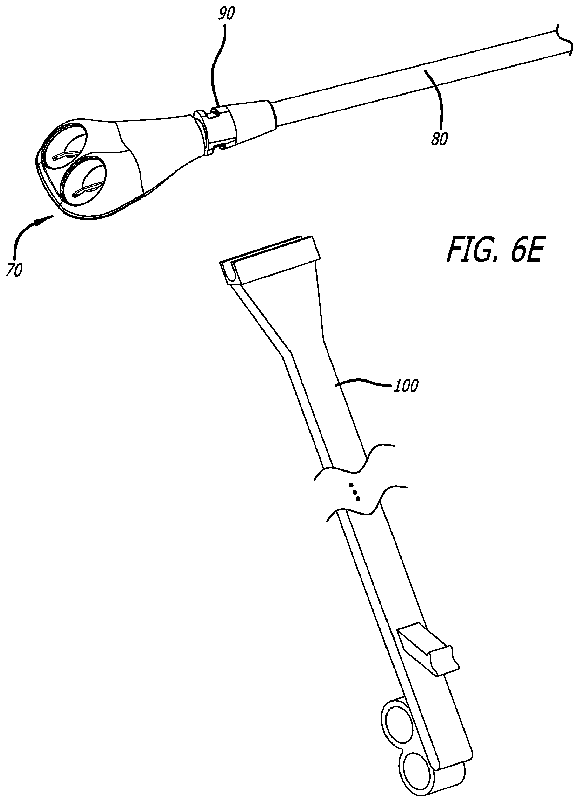

As shown in FIG. 6E, with the cathlock 90 in place, locking the catheter 80 to the port, the user can then actuate, or release, the actuator(s) 170 to release one of the catheter 80 or the cathlock 90 and withdraw the tool 100 from the cathlock 90 but urging the cathlock 90 through the opening 124.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A stabilization and trimming tool (100) for coupling a catheter (80) to an access port (70), comprising:
a handle (110) operatively coupled to a cradle (120) and a clamping device (150), wherein the cradle (120) is designed to hold a cathlock (90), and wherein the clamping device (150) is designed to grip a catheter (80); and
a catheter cutting mechanism (160) coupled to the handle, wherein the catheter cutting mechanism is configured to be actuated following stabilization of the cathlock (90) and the catheter (80) by the handle (110).

2. The stabilization and trimming tool according to claim 1, wherein a first pressure applied to the handle (110) applies pressure to the cradle (120), and wherein a second pressure greater than the first pressure applies pressure to both the cradle (120) and the clamping device (150).

3. The stabilization and trimming tool according to any of claims 1-2, wherein the cradle (120) defines a channel (122), an inner surface (126) of the channel (122) defines a profile that mirrors an outer profile of the cathlock (90).

4. The stabilization and trimming tool according to claim 3, wherein the inner surface (126) of the channel (122) defines a tapered shape.

5. The stabilization and trimming tool according to claim 3, wherein the inner surface (126) of the channel (122) defines a stepped portion configured to abut against a stepped portion (94) of the cathlock (90).

6. The stabilization and trimming tool according to any of claims 1-5, wherein the clamping device (150) includes a pair of clamping members (150A, 150B) covered by a rubber or silicone material.

7. The stabilization and trimming tool according to any of claims 1-6, wherein the catheter cutting mechanism (160) is actuated by an actuator (170).

8. The stabilization and trimming tool according to any of claims 1-7, wherein the cradle (120) includes a pair of jaws (134) transitionable between an open position and a closed position, the jaws (134) configured to retain the cathlock (90) within the cradle (120) in the closed position.

9. The stabilization and trimming tool according to claim 8, wherein a first jaw (134A) of the pair of jaws (134) is rotatably coupled to a first edge of the cradle (110) and a second jaw (134B) of the pair of jaws (134) is rotatably coupled to a second edge of the cradle.

10. The stabilization and trimming tool according to any of claims 1-9, wherein the cradle (110) defines a channel (122) and includes a pair of grips (136) transitionable between a retracted position and an extended position, the grips (136) configured to retain the cathlock (90) within the cradle (110) in the extended position.

11. The stabilization and trimming tool according to claim 10, wherein the pair of grips (136) are slidably engaged with the cradle (110) along an axis extending perpendicular to an axis of the channel (122).

## Patentansprüche

1. Stabilisierungs- und Trimmwerkzeug (100) zum Koppeln eines Katheters (80) an einen Zugangsport (70), umfassend:
einen Griff (110), der betriebsfähig mit einer Halterung (120) und einer Klemmvorrichtung (150) gekoppelt ist, wobei die Halterung (120) dazu ausgelegt ist, einen Katheterverschluss (90) zu halten, und wobei die Klemmvorrichtung (150) dazu ausgelegt ist, einen Katheter (80) zu greifen; und
einen Katheterschneidmechanismus (160), der mit dem Griff gekoppelt ist, wobei der Katheterschneidmechanismus so konfiguriert ist, dass er nach der Stabilisierung des Katheterverschlusses (90) und des Katheters (80) durch den Griff (110) betätigt wird.

2. Stabilisierungs- und Trimmwerkzeug nach Anspruch 1, wobei ein erster Druck, der auf den Griff (110) ausgeübt wird, Druck auf die Halterung (120) ausübt, und wobei ein zweiter Druck, der größer als der erste Druck ist, Druck sowohl auf die Halterung (120) als auch auf die Klemmvorrichtung (150) ausübt.

3. Stabilisierungs- und Trimmwerkzeug nach einem der Ansprüche 1-2, wobei die Halterung (120) einen Kanal (122) definiert, eine Innenfläche (126) des Kanals (122) ein Profil definiert, das ein Außenprofil des Katheterverschlusses (90) spiegelt.

4. Stabilisierungs- und Trimmwerkzeug nach Anspruch 3, wobei die Innenfläche (126) des Kanals (122) eine sich verjüngende Form definiert.

5. Stabilisierungs- und Trimmwerkzeug nach Anspruch 3, wobei die Innenfläche (126) des Kanals (122) einen abgestuften Abschnitt definiert, der so konfiguriert ist, dass er an einen abgestuften Abschnitt (94) des Katheterverschlusses (90) anliegt.

6. Stabilisierungs- und Trimmwerkzeug nach einem der Ansprüche 1-5, wobei die Klemmvorrichtung (150) ein Paar Klemmglieder (150A, 150B) einschließt, die mit einem Gummi- oder Silikonmaterial bedeckt sind.

7. Stabilisierungs- und Trimmwerkzeug nach einem der Ansprüche 1-6, wobei der Katheterschneidmechanismus (160) durch einen Betätiger (170) betätigt wird.

8. Stabilisierungs- und Trimmwerkzeug nach einem der Ansprüche 1-7, wobei die Halterung (120) ein Paar Backen (134) einschließt, die zwischen einer offenen Position und einer verschlossenen Position überführbar sind, wobei die Backen (134) konfiguriert sind, um den Kathodenverschluss (90) innerhalb der Halterung (120) in der verschlossenen Position zu halten.

9. Stabilisierungs- und Trimmwerkzeug nach Anspruch 8, wobei eine erste Backe (134A) des Paares von Backen (134) drehbar mit einer ersten Kante der Halterung (110) gekoppelt ist und eine zweite Backe (134B) des Paares von Backen (134) drehbar mit einer zweiten Kante der Halterung gekoppelt ist.

10. Stabilisierungs- und Trimmwerkzeug nach einem der Ansprüche 1-9, wobei die Halterung (110) einen Kanal (122) definiert und ein Paar Griffe (136) einschließt, die zwischen einer zurückgezogenen Position und einer ausgefahrenen Position überführbar sind, wobei die Griffe (136) so konfiguriert sind, dass sie den Katheterverschluss(90) in der ausgefahrenen Position innerhalb der Halterung (110) halten.

11. Stabilisierungs- und Trimmwerkzeug nach Anspruch 10, wobei das Paar Griffe (136) entlang einer Achse, die senkrecht zu einer Achse des Kanals (122) verläuft, verschiebbar in die Halterung (110) eingreift.

## Revendications

1. Outil (100) de stabilisation et de découpe pour coupler un cathéter (80) à un orifice (70) d'accès, comprenant :
une poignée (110) couplée de manière opérationnelle à un berceau (120) et à un dispositif (150) de serrage, dans lequel le berceau (120) est conçu pour maintenir un cathlock (90), et dans lequel le dispositif (150) de serrage est conçu pour saisir un cathéter (80) ; et
un mécanisme (160) de coupe de cathéter couplé à la poignée, dans lequel le mécanisme de coupe de cathéter est configuré pour être actionné à la suite de la stabilisation du cathlock (90) et du cathéter (80) par la poignée (110).

2. Outil de stabilisation et de découpe selon la revendication 1, dans lequel une première pression appliquée à la poignée (110) applique une pression sur le berceau (120), et dans lequel une seconde pression supérieure à la première pression applique une pression à la fois au berceau (120) et au dispositif (150) de serrage.

3. Outil de stabilisation et de découpe selon l'une quelconque des revendications 1 à 2, dans lequel le berceau (120) définit un canal (122), une surface (126) interne du canal (122) définit un profil qui reflète un profil externe du cathlock (90).

4. Outil de stabilisation et de découpe selon la revendication 3, dans lequel la surface (126) interne du canal (122) définit une forme effilée.

5. Outil de stabilisation et de découpe selon la revendication 3, dans lequel la surface (126) interne du canal (122) définit une partie étagée configurée pour buter contre une partie (94) étagée du cathlock (90).

6. Outil de stabilisation et de découpe selon l'une quelconque des revendications 1 à 5, dans lequel le dispositif (150) de serrage inclut une paire d'organes (150A, 150B) de serrage recouverts d'un matériau en caoutchouc ou en silicone.

7. Outil de stabilisation et de découpe selon l'une quelconque des revendications 1 à 6, dans lequel le mécanisme (160) de coupe de cathéter est actionné par un actionneur (170).

8. Outil de stabilisation et de découpe selon l'une quelconque des revendications 1 à 7, dans lequel le berceau (120) inclut une paire de mâchoires (134) transitoires entre une position ouverte et une position fermée, les mâchoires (134) étant configurées pour retenir le cathlock (90) à l'intérieur du berceau (120) dans la position fermée.

9. Outil de stabilisation et de découpe selon la revendication 8, dans lequel une première mâchoire (134A) de la paire de mâchoires (134) est couplée en rotation à un premier bord du berceau (110) et une seconde mâchoire (134B) de la paire de mâchoires (134) est couplée en rotation à un second bord du berceau.

10. Outil de stabilisation et de découpe selon l'une quelconque des revendications 1 à 9, dans lequel le berceau (110) définit un canal (122) et inclut une paire de pinces (136) transitoires entre une position rétractée et une position déployée, les pinces (136) étant configurées pour retenir le cathlock (90) à l'intérieur du berceau (110) dans la position déployée.

11. Outil de stabilisation et de découpe selon la revendication 10, dans lequel la paire de pinces (136) est engagée de manière coulissante avec le berceau (110) le long d'un axe s'étendant perpendiculairement à un axe du canal (122).
